# EUROPEAN PATENT SPECIFICATION

(11) **EP 0 591 217 B1**
(45) Date of publication and mention of the grant of the patent: **19.04.2000**
(21) Application number: 92903108.6
(22) Date of filing: 27.06.1991
(51) Int. Cl.: A61B 5/08

(54) **OXYGEN CONSUMPTION METER**
MESSEINRICHTUNG FÜR DIE SAUERSTOFFAUFNAHME
APPAREIL DE MESURE DE LA CONSOMMATION D'OXYGENE

(43) Date of publication of application: 13.04.1994
(73) Proprietor: MAULT, James R., Evergreen, Colorado 80439 (US)
(72) Inventor: MAULT, James R., Evergreen, Colorado 80439 (US)
(74) Representative: Moreland, David, Dr.
(86) International application number: US9104587
(87) International publication number: WO9300040

(56) References cited:
- US-A- 2 630 798
- US-A- 3 797 480
- US-A- 3 799 149
- US-A- 4 368 740
- US-A- 4 679 573
- US-A- 4 753 235
- US-A- 4 917 108
- MEDICAL PROGRESS THROUGH TECHNOLOGY, vol.9, no.1, 1982, BERLIN (D) pages 27 - 32 R. SALMINEN ET AL. 'Computerized Breath-By-Breath Analysis of Respiratory Variables During Exercise'
- BRITISH JOURNAL OF ANAESTHESIA, vol.49, 1977, LONDON (GB) pages 575 - 587 J. A. BUSHMAN ET AL. 'Closed circuit anaesthesia'

## Description

### Field of the Invention

This invention relates to indirect calorimeters for measuring metabolic rate on the basis of respiratory oxygen consumption and mare particularly to such a calorimeter which scrubs the CO₂ from the exhaled gas and computes the difference between the inhaled gas volume and the volume of the scrubbed exhaled gas to calculate oxygen consumption.

### Background of the Invention

Measurement of the energy expenditure of humans is important for a number of reasons. For nutritional purposes, measurement of the resting energy expenditure is important for determination of the proper caloric content for feedings of hospitalized patients in view of the fact that certain diseases and traumas may cause the resting energy expenditure to vary substantially from normal values. In burn patients the metabolic rate may increase by as much as 300%. Other hospital situations in which the measurement of rate of metabolic oxygen consumption is important include the adjustment of parental feedings for infants, and the control of respiratory gases during surgical operations. The resting energy expenditure may also decrease substantially in the courts of a weight loss diet, and knowledge of this basal energy requirement is important to the adjustment of caloric inputs in order to achieve a target loss. Similarly, knowledge of caloric expenditure and oxygen consumption during exercise are useful for cardiac rehabilitation and athletic training.

A variety of indirect calorimeters for measuring oxygen consumption during respiration have been devised and are available commercially. These broadly include closed circuit devices wherein oxygen depleted during respiration is replenished from an oxygen source and the volume of replenishing oxygen is measured to determine respiratory oxygen consumption. A device of this type is disclosed in U.S. Patent No. 4,753,245. Open circuit devices generally measure the volume of inhaled gas and the proportions of carbon dioxide and oxygen in exhaled gas to determine the respiratory oxygen consumption. Devices of this class are disclosed in U.S. Patent Nos. 3,523,529, 4,619,269, 4,221,224 and 4,572,208.

All of these devices are relatively complex and expensive and require specially trained technicians for their operation. Their use has largely been limited to hospital settings for the adjustment of nutritional requirements for critically ill patients in intensive cars units.

A potentially simpler and less expensive form of calorimeter would measure the inhaled gas volume, pass the exhaled gas aver a carbon dioxide scrubber to remove the lung contributed CO₂ from the exhaled gas and than measure the remaining gas volume. The difference between the two measured volumes would be a direct function of the respiratory oxygen consumption. However, because the exhaled gas has substantially different temperature and water vapor content than the inhaled gas, the volume measurements may grossly misestimate the actual oxygen consumption. Additionally, because such a device would measure variations in the relatively small differential between two large measurements, design of the device to attain a reasonable accuracy presents a problem.

My U.S. Patent No. 4,917,108 discloses an indirect calorimeter for calculating the metabolic rate of a subject by measuring the oxygen consumption during respiration over a period of time includes a gas flow meter providing output electric signals to a microprocessor which drives a display and printer. A carbon dioxide scrubber is connected to the flow meter and a respiratory connector including a mouthpiece so that inhaled gas passes first through the scrubber and then through the flow meter before being provided to the subject's respiratory system through the mouthpiece. The exhaled gas passes through the scrubber and then through the flow meter. The difference in volume between the inhaled gas and the exhaled gas is proportional to the oxygen consumption of the subject and the microprocessor integrates that signal over the time of the test, and multiplies it by a constant to provide a metabolic rate display. By passing both the inhaled and exhaled gases through the scrubber before their volume is measured, their temperature and humidity are modified to a state of equal temperature and humidity.

### Summary of the Invention

The present invention is accordingly directed toward a simple and low cost indirect calorimeter, or oxygen consumption meter that overcomes the deficiencies of the prior art and may be used by relatively untrained personnel so that it is adapted to use in a wide variety of situations for measurement of oxygen consumption and energy expenditure. The calorimeter of the present invention can operate from atmospheric air or any other oxygen source, such as a mechanical ventilator, and utilizes a chemical carbon dioxide scrubber, and inhaled and exhaled air volume flow meter means. The scrubber, the flow meter, a source of gas to be inhaled and a respiratory connector such as a mask, endotracheal tube or mouthpiece are interconnected with conduits and one-way valves so that inhaled air is first conditioned to adjust its temperature to a level comparable to that of exhaled air, its volume is then measured and it is then passed to the mouthpiece, and exhaled air is passed through a Co₂ scrubber before its volume is measured. The flow meter means preferably provides pulsed signals with each electric pulse representing an increment of flow volume, and the system includes a microprocessor-based computation and display unit which receives the pulse signals, distinguishes inhalations from exhalations, and generates integrals of their differences over a period of time. The microprocessor preferably stores these integrated different signals for short periods of time representing increments of use of the calorimeter during the test and at the conclusion of the test displays the value representing the oxygen consumption over the latter portion of the test, multiplied by a constant to arrive at the display or kilocalories per 24 hour time. This arrangement discards the initial readings which may be inaccurate until the scrubber chemicals have built up a stable temperature and water vapor level. The integration of a large number of respiration cycles minimizes error resulting from limited repeatability of the flow sensor.

A preferred embodiment or the invention, which will subsequently disclosed in detail, employs a single flow meter for measuring both the inhaled and exhaled volume, thereby eliminating a possible differential of accuracy between separate meters as an error source. The flow meter, may be of differential pressure type or may take other forms such as turbine, ultrasonic or electromagnetic meters. The flow meter is connected to receive the air to be inhaled. Conduits and one-way valves connect the mouthpiece to both the inlet of the scrubber and the output of the flow meter. When the user inhales, atmospheric air or air from a mechanical respirator ventilator is drawn into the calorimeter, heated, and passed through the flow meter, to the mouthpiece or other patient interface. Exhaled air passes through the scrubber and the flow meter to the atmosphere or back to the ventilator. The microprocessor analyses the train of pulse it receives from the flow meter to distinguish inhalations from exhalations. In an alternative embodiment, the system includes a flow direction sensor disponed in one the conduits and connected to the microprocessor can distinguish inhalations and exhalations from the flow direction of the air that passes over the direction sensor.

The present calorimeter may also be used in positive pressure ventilation systems using any concentration of oxygen source. This includes intensive care mechanical ventilators and closed loop anaesthesia. The present invention may also be applied to exercise stress testing. In an alternative embodiment of the invention separate flow sensors may be provided for the inhaled and exhaled volume, eliminating the need for the microprocessor to distinguish pulse trains associated with the inhaled and exhaled breaths.

The present invention may adjust the temperature and water vapor content of the inhaled air to substantially that of the exhaled air so that the inhaled volume and the exhaled volume, as measured by the flow meter, may be directly compared to determine the oxygen volume utilized by the patient.

This is achieved through use of a heater to raise the temperature of the inhaled air to substantially the temperature of the exhaled air and passes this heated air directly to the flow meter so that only the exhaled air poses through the scrubber. This embodiment also incorporates means to essentially equalize the water vapor content of the air to be inhaled and the exhaled air. This device preferably takes the form of a water absorbent filter media, such as a sponge, which absorbs the moisture vapor in the exhalation and has its moisture content reduced by the relatively dry air during inhalation. Such devices are used in other respiratory products and are termed "artificial noses". This device may act as a filter for the mouthpiece, passing the inhaled volume to the patient and accepting the exhaled volume for passage to the other apparatus.

For use of the calorimeter in a clinical setting it is desirable that those portions of the apparatus which may be contaminated in use be made disposable. These include the mouthpiece, the CO₂ scrubber, the interconnecting tubing and valving. The flow meter is relatively expensive and economics may prohibit its disposal. Accordingly, in one embodiment of the present invention, one or more disposable bacterial filters are interposed between the flow meter which is contaminated by a patient's breath, and the non- disposable elements, which are protected from the contamination by the filters.

The device of the present invention is thus simple, inexpensive, easy to use, and has excellent accuracy.

### Brief Description of the Drawings

Other objectives, advantages, and applications of the present invention will be made apparent by the following detailed description of a preferred embodiment of the invention. The description makes reference to the accompanying drawings in which:
FIGURE 1 is a perspective view of a preferred embodiment of the indirect respiratory calorimeter of the present invention being used by a subject;
FIGURE 2 is a flow diagram illustrating the manner of operation of the microprocessor used with the present invention;
FIGURE 3 is a partial schematic view of a section of an alternative construction of a single flow meter indirect calorimeter of the type illustrated in Figure 1 but incorporating a mechanical sensor for flow direction to generate an electric signal distinguishing flow associated with exhalations from flows representative of inhalations;
   and
FIGURE 4 is a schematic diagram of a preferred embodiment of the invention in which only the exhaled air and not the inhaled air is passed through the scrubber and the inhaled air is preheated to a temperature comparable to that of the exhaled breath.

### Detailed Description of the Preferred Embodiment

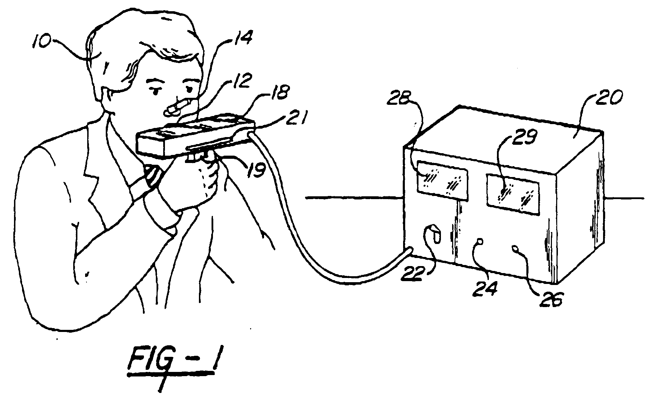

Figure 1 illustrates a preferred embodiment of the present invention in use. A user 10 inhales and exhales air from and into a user respiratory interface taking the form of a mouthpiece 12 adapted to engage the inner surfaces at the user's mouth so as to form the sole passage for inhaled and exhaled air passing through the mouth. A nose clamp 14 of conventional construction may be employed in connection with the mouthpiece 12 to assure that all respiratory air passes through the mouthpiece. In alternative configurations a mask that engages the nose as well as the mouth might be employed. In embodiments intended for use with mechanical ventilators, an endotracheal tube may be employed rather than a mouthpiece or mask.

The mouthpiece 12 is directly connected to a small instrument housing 18 that is manually held by a handle 19 fixed to the housing. The instrument housing 18 contains the other components of the system and is connected to a microprocessor-based computation and display unit 20. The unit 20 includes an ON/OFF switch 22 and a pair of LED signal lights 24 and 26. The signal light 24 is illuminated when the switch is first thrown to the ON position to indicate that the test is underway. At the end of a predetermined time, the light 24 is extinguished and the signal light 26 is illuminated, signaling that enough time has elapsed and that the subject may quit at any time. In the preferred embodiment of the present invention, the housing 18 and the mouthpiece 12 arm intended for one use and are disposable.

The unit 20 also includes a first digital display 28 which displays the value VO₂, the volume of inspired oxygen consumed per minute, and a second display 29 which exhibits the value of Kcal/24 hours, i.e., the value arrived at by multiplying the integral of the difference between the inhaled volume and the scrubbed exhaled value by a constant.

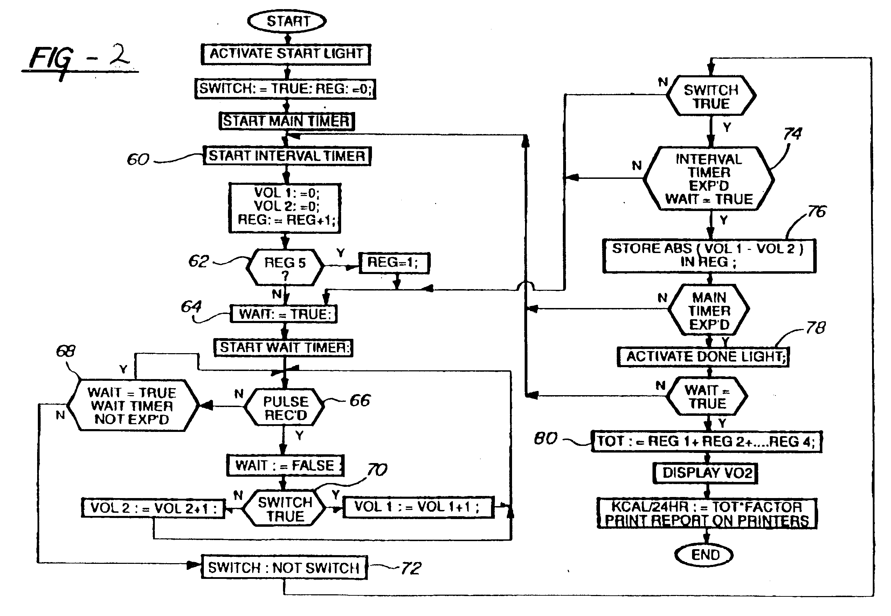

Figure 2 provides a flow diagram illustrating the general flow of operation of the microprocessor in the preferred embodiment of the present invention. The algorithm uses five registers to stare values of integrals of the volumes as generated by the flow meter 42 during five successive intervals.

First, the LED signal light 24 of Figure 1 is illuminated in order to indicate to the user that the test is underway. The boolean variable SWITCH is then initialized to true and the integer variable REG, for keeping track of the current register in use, is initialized to 0.

Next, the main timer is started. This timer expires after the minimum time required for the test has passed. In the preferred embodiment, this tine is 10 minutes.

Next, at the step indicated at 60, an interval timer is started. This timer keeps track of short increments of the total time. In the preferred embodiment this time is 1 minute. The microprocessor finds the integrated difference between the exhaled and inhaled gas volumes for each increment of time and stores these values at memory locations designated REG 1, REG 2... The processor only stores the most recent 5 periods, so that the initial readings that may be inaccurate due to temperature differences between the inhaled and exhaled gas are discards. Next, the counter variables Vol 1 and Vol 2 are initialized to 0, and REG is incremented by one. At 62 a check is made as to whether the current register is greater than 5. It not, the algorithm goes to the step indicated at 64. If REG > 5, then REG is set back to 1 and the algorithm continues at step 64.

At 64, the boolean variable WAIT is set to true. This variable is used to indicate whether the microprocessor is waiting for a pulse signal from the flow meter. Next, a wait timer is started. This timer is used to create a maximum time in which the microprocessor will wait for a pulse signal. Next, the algorithm goes to the step indicated at 66.

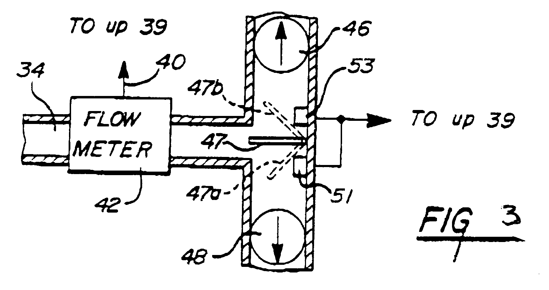

At 66, a check is made as to whether a pulse signal has been received from the flow meter. If not, the algorithm goes to the step indicated at 68. It a pulse has been received at 66, WAIT is set to false and at step 70 a check is made as to whether SWITCH is true. If it is, Vol 1 is incremented. If it is not, Vol 2 is incremented. In either case, the algorithm then goes back to step 66. In the alternative embodiment at Figure 3 utilizing the flow direction sensor switches 51 and 53, no SWITCH variable is needed for the algorithm. In this embodiment the microprocessor may check the signal being received from the flow direction sensors 51 and 53 and then increment Vol 1 if the user is inhaling or Vol 2 if he is exhaling.

At 68, a check is made as to whether both WAIT is true and the wait timer has not expired. If either of these conditions are not true, the algorithm goes to the step indicated at 72. Else, the algorithm continues at step 66.

At 72, SWITCH is complemented. Next, a check is made as to whether SWITCH is set to true. If so, the algorithm goes to the step indicated at 74. If SWITCH is false, the algorithm goes back to step 64.

At 74, a check is made as to whether either the interval timer has expired or WAIT is set to true. IF either of these conditions is true, the algorithm continues to the step indicated at 76. Else, it goes back to step 64.

At 76, the absolute value of the difference between Vol 1 and Vol 2 (the difference between exhaled and inhaled oxygen volumes) is stored in the current register. Next, a check is made as to whether the main timer has expired. If so, the algorithm continues to the step indicated at 78. Else, the algorithm goes back to step 60 and goes through the routine another time, loading the integrals of the volume signals for the next interval of time (preferably one minute) into the next register. At 78, the LED signal light 26 of Figure 1 is illuminated in order to indicate to the user that he may quit at any time. The microprocessor senses that the user quit when no pulse signal is received from the flow meter for an entire period of the wait timer.

Next, a check is made as to whether WAIT is set to true. If so, the algorithm continues at the step indicated at 80. IF WAIT is false, the algorithm goes back to step 60. At 80, the five registers are summed together to arrive at an integral of the difference between exhaled and inhaled oxygen volumes over the latest portions of the test. Next, the volume of the oxygen inspired per minute, VO₂, is displayed on the digital display 28 of the microprocessor unit 30, as shown in Figure 1. Finally, the sum is multiplied by a factor to arrive at the number of kilocalories that the subject expends during a 24 hour period. This factor is arrived at as follows: Approximately 5 kilocalories are expended for every 1 liter of oxygen consumed in a minute. In the preferred embodiment of the present invention, the volumes of oxygen are measured in millimeters. Therefore, the resting energy expenditure (REE) may be calculated from the Weir equation, set forth subsequently. This result is displayed on the digital display 29 of the unit 20, and a report of the test results is outputted to a printer. An alternative algorithm may also display the volume of oxygen inspired per minute after the main timer has expired and continue to update it after each interval period.

The disclosure of the algorithm depicted in Figure 2 is not intended to limit the present invention. Many different algorithms may be implemented to achieve the same results. For the purposes of illustration, well-known housekeeping functions, such am error checking features, were omitted from the algorithm of Figure 2.

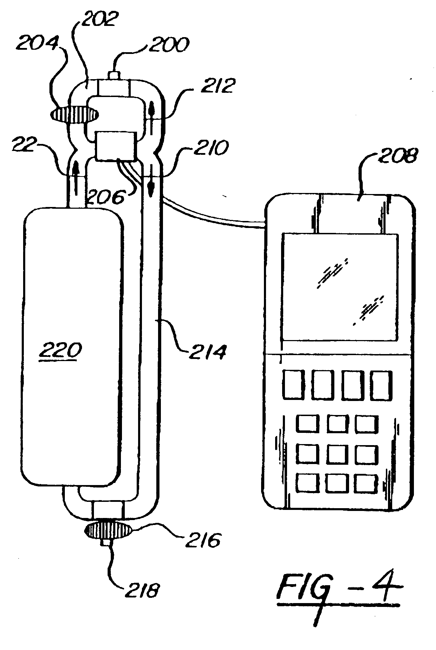

In an embodiment of the invention, illustrated in Figure 4 the inhaled air is not passed through the CO₂ scrubber prior to provision to the patient's mouthpiece. The CO₂ scrubber only receives exhaled air.

Referring to Figure 4 an air source 200, which may represent a connection to ambient air or a positive pressure ventilator, connects to a conduit 202 which passes the air through a preheater 204 which is preferably an electric resistance heater. The heater raises the temperature of the incoming air to approximately 34 degrees Centigrade, which will be within a degree or two of the temperature of the exhaled air. Alternatively, a sensor (not shown) may measure the exhaled air temperature and control the temperature of the preheater 204. The heated inhaled air is then passed through the flow meter 206 which provides its electric output to the microprocessor based computer 208. A pair of unidirectional flow valves 210 and 212 direct the preheated inhaled air through a conduit 214 to a moisture absorbing filter 216 of the type employed with "artificial noses". The filter essentially constitutes moisture absorber, such as a filter formed of fibrous elements or a sponge. The artificial nose 216 directs the inhaled air to the patient's mouthpiece 218.

When the patient exhales through the mouthpiece the exhalation is first passed through the artificial nose 216. This adjusts its water vapor content to a level comparable to that of the inhaled air. The artificial nose thus absorbs moisture from the exhaled breath and has its moisture level reduced by the incoming breath. The result is to effectively reduce the moisture vapor content of the exhalation. After passing through the artificial nose, the exhaled air passes through a CO₂ scrubber 220 and the output of the scrubber is directed to the flow meter 206 by a one-way valve 222. The output or the flow meter is returned the air source 200 by the one-way valve 212.

In this configuration, on an inhalation, the patient receives a fresh breath of air. In the previous configurations, since the inhaled air came from the CO₂ scrubber, there was a need to minimize the volume of the scrubber to in turn minimize the rebreathing of the previously exhaled air on a subsequent inhalation. The removal of this constraint allows the use of a substantially larger CO₂ scrubber.

## Claims

1. An indirect calorimeter, operative to measure the respiratory oxygen consumption per unit time of a subject, comprising a respiratory connector (12) operative to be supported in contact with a subject to pass respiratory gases as the subject breathes into said respiratory connector, a source of respiratory gases (200), a scrubber 220 having a gas inlet and gas outlet and being operative to absorb carbon dioxide from said gases passing between its inlet and outlet, a gas flow meter (206) operatively connected to said respiratory connector operative to generate signals as function of the volume of the gases passing through the gas flow meter, valves (22), (212) and conduits (202), (214) interconnecting the respiratory connector, scrubber and gas flow meter, means (208) for receiving output signals from the gas flow meter and for generating a signal proportional to the integral of the differences between the inhaled and exhaled gas volumes over a period of time:
the valves and conduits being so arranged that upon the subject inhaling gases are caused to pass directly through the gas flow meter and then the subject's respiratory system through the respiratory connector without passing through the scrubber, and upon the subject exhaling the exhaled gases are caused to pass from the respiratory connector first through the scrubber, then through the gas flow meter, whereby the signal proportional to the integral of the differences between the inhaled and exhaled gas volumes are a measure of the subject's respiratory oxygen consumption.

2. The indirect calorimeter of claim 1, further including a temperature conditioner (204) connected between said source of respiratory and said gas flow meter.

3. The indirect calorimeter of claim 1 further including bacterial filter means (204) interconnected by conduits between said respiratory connector and the gas flow meter to prevent the flow of bacteria from the subject to the flow meter.

## Patentansprüche

1. Indirektes Kalorimeter, das dazu geeignet ist, den Atmungssauerstoffverbrauch pro Zeiteinheit einer Person zu messen, umfassend eine Atmungsanschlußvorrichtung (12), die in Kontakt mit einer Person gehalten wird, um Atemgase weiterzuleiten, wenn die Person in die Atmungsanschlußvorrichtung ausatmet, eine Quelle der Atmungsgase (200), einen Gasreiniger (220) mit einem Gaseinlaß und einem Gasauslaß, der dazu geeignet ist, Kohlendioxid von den Gasen zu absorbieren, die zwischen dem Einlaß und dem Auslaß hindurchgeführt werden, ein Gasdurchflußmeßgerät (206), das auf operative Weise mit der Atmungsanschlußvorrichtung verbunden ist und dazu geeignet ist, Signale als Funktion des Volumens der Gase, die durch das Gasdurchflußmeßgerät hindurchgeführt werden, zu erzeugen, Ventile (22), (212) und Leitungen (202), (214), welche die Atmungsanschlußvorrichtung, den Gasreiniger und den Gasdurchflußmesser miteinander verbinden, ein Mittel (208) zum Empfangen von Ausgangssignalen vom Gasdurchflußmeßgerät und zum Erzeugen eines Signals, das proportional zum Integral der Differenz zwischen dem eingeatmeten und dem ausgeatmeten Gasvolumen über eine Zeitperiode hinweg ist;
wobei die Ventile und Leitungen so angeordnet sind, daß, wenn die Person einatmet, Gase direkt durch das Gasdurchflußmeßgerät und dann durch die Atmungsanschlußvorrichtung in das Atmungssystem der Person fließen, ohne dabei durch den Gasreiniger hindurchzutreten, und, wenn die Person ausatmet, die ausgeatmeten Gase von der Atmungsanschlußvorrichtung zuerst durch den Gasreiniger und dann durch das Gasdurchflußmeßgerät geführt werden, wobei das Signal, welches proportional zum Integral der Differenz zwischen dem eingeatmeten und dem ausgeatmeten Gasvolumen ist, ein Maß für den Atmungssauerstoffverbrauch der Person darstellt.

2. Indirektes Kalorimeter nach Anspruch 1, weiters umfassend ein Temperaturkonditionierungsgerät (204), das zwischen der Quelle der Atmungsgase und dem Gasdurchflußmeßgerät angeschlossen ist.

3. Indirektes Kalorimeter nach Anspruch 1, weiters umfassend ein Bakterienfiltermittel (204), das durch Leitungen zwischen der Atmungsanschlußvorrichtung und dem Gasdurchflußmeßgerät angeschlossen ist, um den Durchfluß von Bakterien von der Person zum Durchflußmeßgerät zu verhindern.

## Revendications

1. Calorimètre indirect, capable de mesurer la consommation d'oxygène respiratoire d'un sujet par unité de temps, comprenant un raccord respiratoire (12) pouvant être supporté en contact avec un sujet pour envoyer les gaz respiratoires au fur et à mesure que le sujet respire dans ledit raccord respiratoire, une source de gaz respiratoires (200), un laveur (220) ayant une entrée de gaz et une sortie de gaz et qui est capable d'absorber le dioxyde de carbone desdits gaz qui passent entre son entrée et sa sortie, un débitmètre de gaz (206) relié de façon à fonctionner pour générer des signaux en fonction du volume des gaz qui traversent le débitmètre de gaz, des vannes (22), (212) et des conduites (202), (214) reliant le raccord respiratoire, le laveur et le débitmètre de gaz, un moyen (208) pour recevoir des signaux de sortie du débitmètre de gaz et pour générer un signal proportionnel à l'intégrale des différences entre les volumes de gaz inhalés et exhalés sur une période de temps :
les vannes et les conduites étant disposées de telle sorte que, lorsque le sujet inhale, elles provoquent le passage des gaz directement à travers le débitmètre de gaz et ensuite le système respiratoire du sujet par l'intermédiaire du raccord respiratoire sans qu'ils traversent le laveur, et que, lorsque le sujet exhale, elles provoquent le passage des gaz exhalés depuis le raccord respiratoire d'abord à travers le laveur, puis à travers le débitmètre de gaz, de telle sorte que le signal proportionnel à l'intégrale des différences entre les volumes des gaz inhalés et exhalés constitue une mesure de la consommation d'oxygène respiratoire du sujet.

2. Calorimètre indirect suivant la revendication 1, comprenant de plus une unité de conditionnement de la température (204) raccordée entre ladite source de gaz respiratoires et ledit débitmètre de gaz.

3. Calorimètre indirect suivant la revendication 1, comprenant de plus un moyen de filtration des bactéries (204) relié par des conduites entre ledit raccord respiratoire et le débitmètre de gaz pour empêcher le passage des bactéries depuis le sujet vers le débitmètre de gaz.
